(19) Europäisches Patentamt
European Patent Office
Office européen des brevets

(11) **EP 2 166 998 B1**

## (12) EUROPEAN PATENT SPECIFICATION

(45) Date of publication and mention
of the grant of the patent:
**09.12.2015 Bulletin 2015/50**

(21) Application number: **08784524.4**

(22) Date of filing: **20.06.2008**

(51) Int Cl.:
*A61F 5/48* (2006.01)     *A47C 21/06* (2006.01)
*A61F 13/15* (2006.01)

(86) International application number:
**PCT/EP2008/004967**

(87) International publication number:
**WO 2008/155126 (24.12.2008 Gazette 2008/52)**

(54) **LIQUID ABSORBENT SURFACE PAD**

PAD MIT FLÜSSIGKEITSABSORBIERENDER OBERFLÄCHE

article ä surface absorbente de liquide

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **21.06.2007 EP 07012156**

(43) Date of publication of application:
**31.03.2010 Bulletin 2010/13**

(73) Proprietor: **ALBIS INTERNATIONAL S.R.L.
Milano (IT)**

(72) Inventors:
• **DI GIROLAMO, Remo
I-65125 Pescara (IT)**
• **BOSCOLO, Galliano
I-13856 Vigliano Biellese (IT)**

(74) Representative: **Matkowska, Franck et al
Matkowska & Associés
9, rue Jacques Prévert
59650 Villeneuve d'Ascq (FR)**

(56) References cited:
**US-A- 4 844 965          US-A1- 2002 034 914
US-A1- 2004 121 683     US-B1- 6 238 379**

## Description

## Technical field

The present invention relates to a novel surface pad having liquid absorbent properties, and more particularly to an absorbent underpad used in the field of adult incontinence, such as bed pads used in the bed of adult incontinent to protect the mattress against soilage, or cover pads used for protecting a chair, armchair or wheelchair.

## Prior Art

In the particular field of adult incontinence, the protection against urine and faeces losses for severe or moderate adult incontinence is normally satisfied by means of personal absorbent pads, such as diapers, worn by the adult incontinent. In addition to the use of such personal absorbent pads, it is however necessary to use bed pads for protecting the bed mattress of an adult incontinent Such bed pads are useful in case of failure of the personal absorbent pad worn by the adult incontinent, or when the personal absorbent pad worn by the adult incontinent is being changed, or during toileting and therapy phases of the adult incontinent. For the same reason, it is also common to use cover pads for protecting a chair, armchair or wheelchair of an adult incontinent.

In use a key function of the bed pads is to collect and absorb the limited amount of solids and liquids eventually lost by the main absorbent product worn by the incontinent or the limited amount of liquids or other means that may reach the bed surface during toileting or therapy procedures of the adult incontinent. The bed pad, as said, is not worn by the incontinent, but is positioned over the bed in the area where the adult incontinent normally lays with the part of his body worn with the personal absorbent product To ensure sufficient protection, the bed pads are pretty wide products: the most used sizes are about 60x90 cm and 80x180 cm wide. The absorbent part extension of the latter (i.e. 80x180 cm wide) is similar to the one of the 60x90 cm product, but it has also two not-absorbent side flaps that are foldable under the mattress. One of the key bed pad's product requirement is to provide a very uniform absorbency performance in the widest possible product area to ensure the absorbency and the bed protection even when the incontinent moves in the bed towards the edges of the bed pad. Given the limited amount of liquids to be absorbed by a bed pad, differently from the personal absorbent product worn by the incontinent, a bed pad actually only needs a limited absorbency capacity but, importantly, evenly spread all over the product. Further, in the real use the bed pad is heavily and intensely stressed from a mechanical stand point by the actions exerted by the adult incontinent body weight and movements. To work properly in the real conditions, the bed pad should keep its structure very stable and its integrity for a long period of time, either when it becomes wet or dirty (e.g. by failures of the worn absorbent product), or even when the worn absorbent product doesn't leak and the bed pad is not soiled and remains dry, thereby increasing the bed pad life and avoiding unnecessary bed pads waste.

US patent N°4,844,695 discloses a liquid absorbent surface pad having the characteristics defined in the preamble of claim 1.

Absorbent underpads, in particular bed pads, that are currently commercially available in the market, have almost always the same structure disclosed for example in US patent application 2001/0031515 or in European patent application EP-A-0 556 996. This structure comprises a liquid permeable topsheet and a liquid impervious backsheet that are glued together along their side edges, and an absorbent core layer positioned between the said topsheet and backsheet. Most often, the liquid permeable topsheet is a nonwoven layer such as for example a spunbonded layer and the liquid impervious backsheet is a plastic film, in particular a polyethylene film. The liquid absorbent core layer can have different structures, but the most widely used absorbent core layer is made of cellulose fluff adhered to the topsheet and backsheet by glue strips.

The industrial process of making absorbent underpads with an absorbent core layer comprising cellulose fluff is the following. Cellulose pulp is defibrated in a mill station. The fluff coming out from the mill is layed down in a drum that releases formed fluff cores spaced out one to the other onto a strips glued impervious backsheet. The liquid permeable topsheet, having glue strips on one side, is laid down with the glued side over the fluff core and the laminate (liquid permeable topsheet / liquid absorbent core/ liquid impervious backsheet) is calendered, then folded in the machine direction (MD), cut in the cross direction (CD) into separated single pads, that are then folded in cross direction (CD), staked and packed.

Such industrial process has some limitations. In the current industrial conditions, it is very difficult to make a thin absorbent fluff core layer having a basis weight lower than 140-150 gsm at a reasonable production speed and without significant and detrimental formation defects in the core layer (not homogenous fluff distribution, formation of holes).This industrial process has also speed limitation mostly caused by the technical problems related to the handling of a quite light and wide cellulose fluff core layer in the formation, lay down and transport production line sections. In practise, the typical maximum line speed is 130 -160m/min. Current products have the following typical main features: 1) thickness ranging from 2.16 to 2.98 mm measured at 0.5 KPa ; 2) MD tensile strength of the laminate products ranging from 9.18 N/cm to 13.7 N/cm.

The absorbent underpad having the aforesaid known structure has also some disadvantages. In use, the homogeneity of the liquid absorbent core layer is quickly altered, in particular under the repeated contacts with the mobile adult body, and despite the glue strips presence both on the nonwoven liquid permeable topsheet and on the impervious backsheet plastic film. The fluff core adhesion to the glue strips is in fact only partial: it is a core's surface fibers contact phenomenon that does't stabilize intimately the cellulose fibers inside the fluff core thickness where they remain basically free to move; so it doesn't allow, in the fluff core area, a significant bridge effect and stabilizing synergy between the nonwoven liquid permeable topsheet and the plastic film impervious backsheet For this reason, the cellulose fluff core, under the mentioned in use stressing conditions, can easily loose its relatively uniform initial distribution, quickly leading to very heterogeneous absorbent material distribution since wide parts of the absorbent core surface become free of fluff fibres, and then the nonwoven topsheet and plastic backsheet get directly in contact there. Consequentlly, wide parts of the bed pad product are not capable anymore to absorb liquids, while other parts of it have surplus of absorbent fluff core material. This leads to poor in use product performance and to very low comfort level. Moreover, in some cases, the topsheet and backsheet may be accidentally separated along the side edges, thereby prejudicially freeing the absorbent fluff. Furthermore, to enable the automatic gluing operation of the top sheet with the backsheet in a normal production process, the manufacturer is obliged to oversize both the backsheet and the topsheet, compared to the absorbent core dimensions, in order to allow the product's edges sealing all around the absorbent core and to keep unglued margins along the MD side edges thereof.

In addition, the absorbent pads of the prior art have also the following disadvantages:

- they don't allow a good liquid distribution toward the very wide product surface area;
- the liquid absorbed by the core tends to remain locally concentrated since the cellulose core fibers are very loose and capillarity force is quite week;

As a consequence the absorbed liquid concentration reaches a very high level and quickly saturates the limited wetted area. So liquid easily comes back to the adult incontinent body that lays or seats on it.

### Objective of the invention

The present invention proposes a novel liquid absorbent surface pad, in particular a novel liquid absorbent underpad mostly used in the field of adult incontinence, but also in baby, children and feminine hygiene fields. Said novel liquid absorbent surface pad overcomes the aforesaid drawbacks inherent to the structure and to the manufacturing process of liquid absorbent surface pad of the prior art that are made of at least three layers: a liquid permeable topsheet bonded with a liquid impervious backsheet, and an absorbent core layer positioned between the topsheet and backsheet.

### Summary of the invention

The liquid absorbent surface pad of the invention is defined in claim 1.

The term "surface pad" used therein means any absorbent pad that comprises a liquid impervious backsheet and absorbent means, and that is specifically intended for protecting a surface against liquid soilage. In use, a "surface pad" is laid onto the surface that has to be protected, the liquid impervious backsheet being in contact with the surface. The term "surface pad" does not encompass personal absorbent product that are worn by a user, such as personal hygienic products like diapers, napkins,...

Within the scope of the invention, a preferred surface pad is an underpad, such as a bed pad, that is intended to be positioned underneath a person, typically an adult incontinent, or baby or a children, for protecting a mattress, a chair, ....

The term "laminate" used therein means a multilayer product wherein the layers are strongly and intimately bonded together on their whole surface, in such a way that the laminate can be handled without any risk of accidental delamination of the layers. In addition, the laminate can be also embossed to further increase the laminate strength and its fluid distribution capability.

The terms "pulp layer" used therein and in the claims encompass any absorbent layer essentially comprising pulp. The pulp layer can also contain additional materials such as for example superabsorbent material (SAM) and / or synthetic fibers.

The term "pulp" as used therein and in the claims refers to absorbent material made of or containing fibres, especially short fibres, from natural sources such as woody and non-woody plants. Woody plants include, for example, deciduous and coniferous trees. Non-woody plants include, for example, cotton, flax, esparto grass, milkweed, straw, jute hemp, and bagasse.

Within the scope of the invention, the absorbent pulp layer can be made solely of pulp fibres, but can also be made of a dry mixture of pulp fibres with other materials, provided the said mixture can be dry-laid onto a spunbonded layer, by air-laid techniques or the like.

Preferably, the top nonwoven layer, which is not in contact with the liquid impervious backsheet, is hydrophilic

Preferably, the absorbent laminate of the invention is a hydroentangled laminate.

Preferably the liquid absorbent surface pad of the invention is thin and light, the pulp layer having a basis weight less than 100gsm, and preferably less than 60 gsm.

Preferably, the liquid absorbent surface pad has a thickness less than 1mm measured at a loaded pressure of 0.5KPa.

Preferably, the liquid absorbent pad has a machine direction tensile strength higher than 14 N/cm, and even more preferably higher than 16 N/cm.

Preferably, the liquid absorbent pad has a rewet less than 3g of liquid measured according to the so-called "rewet method", hereafter described in details.

In one variant the absorbent laminate is glued with the liquid impervious backsheet.

In another variant, the absorbent laminate is ultrasonic bonded or thermal bonded with the liquid impervious backsheet.

Another object of the invention is a method of making aforesaid liquid absorbent surface pads. According to this method, a web of an absorbent laminate web as defined above, and a web of liquid impervious backsheet are bonded together and cut into single absorbent pads.

In a particular variant, the web of absorbent laminate and the web of liquid impervious backsheet are preferably manufactured off-line separately.

## Brief description of the drawings

The characteristics and advantages of the invention will appear more clearly on reading the following detailed description which is made by way of non-exhaustive and non-limiting example, and with reference to the accompanying drawings, in which:

- Figure 1 is a schematic view in cross section of an absorbent pad of the invention,
- Figures 2 and 3 show two examples of absorbent bed pad that are used for protecting a bed mattress,
- Figure 4 is a schematic drawing of a continuous system for off-line producing the absorbent laminate,
- Figure 5 is a schematic drawing of a continuous system for making the absorbent pad of figure 1 from a roll of absorbent laminate web and from a roll of liquid impervious web,
- Figure 6 is a schematic view in cross section of a folded liquid impervious web that can be used as backsheet for making the absorbent pad with lateral flaps of figure 3,
- Figure 7 is a photography of a bed pad of the prior art, wetted with a certain amount of blue colored solution, and showing the stain obtained after 1 minute, and
- Figure 8 is a photography of a bed pad of the invention, wetted with the same amount of a blue colored solution as per Fig.7, and showing the stain obtained after 1 minute.

## Detailed description

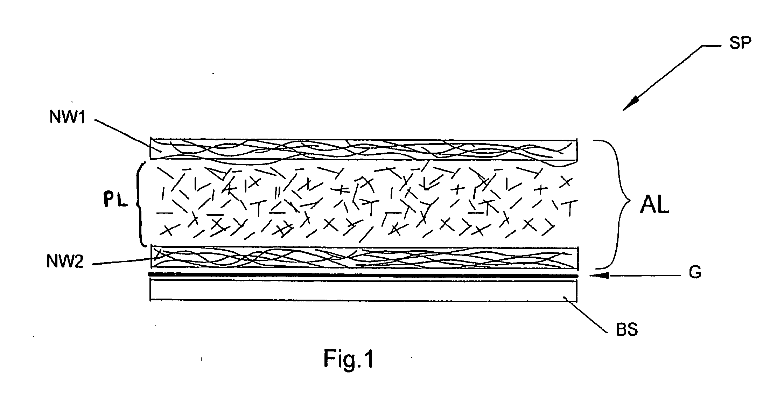

Referring to figure 1, the liquid absorbent surface pad SP of the invention is made of absorbent laminate AL that is bonded with a liquid impervious backsheet BS, for example by means of glue G. Within the scope of the invention, the bonding of the absorbent laminate AL with the liquid impervious backsheet BS can be performed by means of other known techniques, such as for example ultrasonic bonding, heat welding, ...

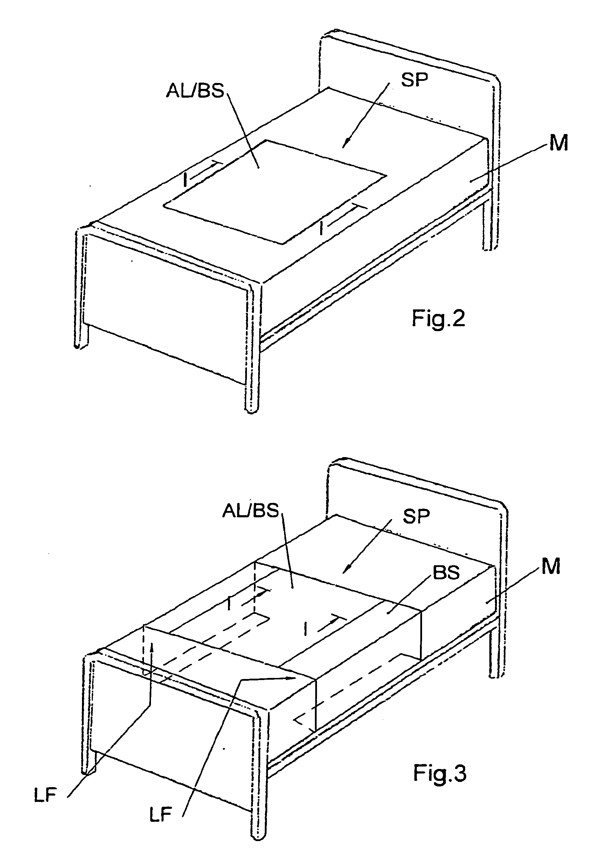

The absorbent surface pad SP is preferably used in the field of adult incontinence as an absorbent underpad that is positioned between the adult incontinent and a surface that needs to be protected against urine and faeces. For example, referring to figures 2 and 3, the absorbent surface pad SP is being used as bed pad for protecting the mattress M of the bed against soilage. When used as bed pad, the liquid impervious backsheet BS is in contact with the bed mattress M and the absorbent laminate AL is on top. In the field of adult incontinence, the absorbent surface pad SP can also be used as underpad for protecting a chair, armchair or wheelchair.

In the particular example of figure 2, the absorbent laminate AL and the backsheet BS have the same dimensions. In the particular example of figure 3, the width dimension of the backsheet BS is greater than the width dimension of the absorbent laminate AL, in order to form, with the backsheet BS, lateral flaps LF that can be fold around the mattress M, while the backsheet BS and the absorbent laminate AL are equal in length.

The invention is however not limited to the field of adult incontinence, and the absorbent surface pad SP can be used more generally for protecting any surface against liquid. For example, it can be used as a cover pad to protect a surface, like for example a table.

The structure of the absorbent surface pad SP and the process for making the absorbent surface pad SP are now going to be detailed.

Structure of the surface pad

Referring to figure 1, the absorbent laminate AL comprises three superposed layers that are strongly and intimately bonded together one absorbent pulp layer PL sandwiched between two liquid permeable nonwoven layers NW1 and NW2.

At least one of the liquid permeable nonwoven layers NW1, NW2 is a spunbonded nonwoven layer, and the other nonwoven layer is a spunbonded nonwoven layer or a carded layer. When priority is given to soft touch for the pad, the top nonwoven layer NW1 will be preferably a carded nonwoven layer. When priority is given to mechanical resistance of the surface pad, especially resistance to abrasion, the top nonwoven layer NW1 will be preferably a spunbonded nonwoven layer.

Preferably, the top nonwoven layer NW1 (spunbonded layer or carded layer) is hydrophilic. The hydrophilic properties can be obtained by applying a hydrophilic solution on the top nonwoven layer (NW1) as described in details hereafter. When the top nonwoven (NW1) is a spunbonded layer, the hydrophilic properties can also be obtained by adding a hydrophilic melt additive in the melt phase of the polymer(s) that are extruded for forming the spun filaments in the spunbonding unit. This melt additive is for example hydrophilic additive Ciba ® IGASURF ™ HL 560 commercialized by CIBA. When the top nonwoven (NW1) is a carded layer, the hydrophilic property can be obtained by adding the hydrophilic melt additive directly in the extruder during the production of the staple fibers that will be fed to the carding machine.

The absorbent pulp layer PL comprises short fibres from natural sources such as woody and non-woody plants. Woody plants include, for example, deciduous and coniferous trees. Non-woody plants include, for example, cotton, flax, esparto grass, milkweed, straw, jute hemp, and bagasse. The pulp layer can also contain additional materials such as for example superabsorbent material (SAM) and / or synthetic fibers.

The three layers NW1, PL, NW2 are bonded together by using any bonding technology known in the field of nonwoven, and including notably: water needling, mechanical needling, thermal bonding, ultrasonic bonding, air trough bonding and chemical bonding. Water needling is however preferably used, the three layers NW1, PL, NW2 being in that case intimately and strongly bonded together by hydroentanglement.

The absorbent laminate AL can also be embossed (e.g. via hydroentanglement) on the top NW1 side or in both sides to further strengthen it and to further enhance its liquid distribution capability.

The absorbent laminate AL can also comprise additional layer(s), such as for example a melt blown layer positioned in-between the absorbent pulp layer PL and the nonwoven layer NW1 or NW2.

The liquid impervious backsheet BS is for example a liquid impervious plastic film, and for example a polyethylene film. This film can be permeable to gas and water vapour. The liquid impervious backsheet BS can also be for example a nonwoven layer coated with a liquid barrier material, such as for example a plastic coating.

Due to the strong bonding of the pulp layer PL with the other nonwoven layers NW1 and NW2, the absorbent surface pad SP exhibits higher integrity and mechanical stability (see table II hereafter). In particular, in use the distribution of the pulp fibres in the absorbent pulp layer is not altered or only slightly altered by repeated handling of the pad or by the repeated contacts with the body of the adult incontinent. The homogeneity of the absorbent pulp layer remains very good even after hours or days of usage. This provides greater comfort level to the user and ensures the availability of the full potential absorbency performance of the product even after hours or days of usage.

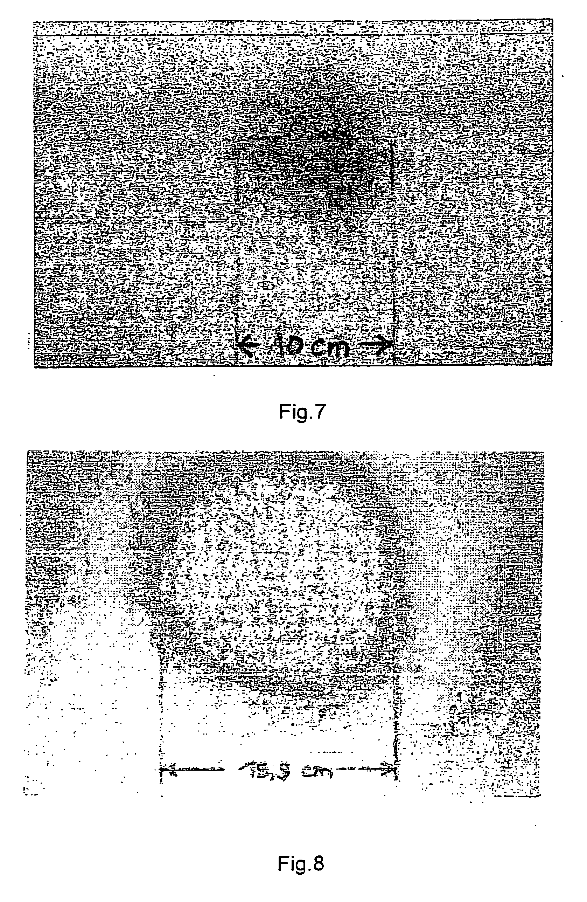

Furthermore, when liquid is absorbed, it spreads fastly on a wider area of the product, compared to the state of art products Therefore the wetted area shows a better dryness thanks to the limited liquid rewetting (see Tables III and IV hereafter and figures 7 and 8).

Advantageously, the laminate AL can also have the same dimensions than the backsheet BS. The absorbent coverage area is thus maximal and can reach the 100% of the total product area for the bed pads without foldable flaps. Currently, with state of art of bed pads, no known product can reach such a maximal absorbent area coverage (at least without extra usage of nonwoven topsheet and plastic backsheet layers and significant process complexity).

The absorbent surface SP is also advantageously thin and light, and thereby very comfortable and less cumbersome. By way of example only, the laminate AL can be made of:

- a 10 gsm spunbonded layer NW1 (or NW2),
- a 45 gsm pulp layer
- a 15 gsm carded layer NW2 (or NW1).

The adhesive layer G can be made of hot melt glue and has for example a basis weight between 0.5-2gsm. The backsheet BS is for example, but not limited to, a polyethylene film having a basis weight between 8-35 gsm and preferably 19-25 gsm.

The use of the absorbent laminate AL provides also advantages for the manufacturing process of the pad SP. As described in more details hereafter, the laminate AL can be advantageously manufactured off-line, and then assembled with the liquid impervious backsheet. Furthermore, the strong bonding of the pulp layer PL with the other nonwoven layers NW1 and NW2 facilitates also the manufacturing process of the pad SP, because there is no pulp loss when cutting the laminate AL.

The bonding of the absorbent laminate AL with the liquid impervious backsheet BS is not necessarily obtained by means of glue, but can be performed by means of other known techniques, such as for example ultrasonic bonding, heat welding, ... More especially, the use of glue can be advantageously avoided and the bonding can be performed only by ultrasonic bonding or thermal bonding, when the nonwoven layer (NW2) in contact with the liquid impervious backsheet BS comprises fibres or filaments comprising a polymer having a similar melting point than the polymeric material of the liquid impervious backsheet BS, more especially when the nonwoven layer (NW2) in contact with the liquid impervious backsheet BS comprises fibres or filaments comprising a polymer that is the same than the polymer of the liquid impervious backsheet BS. For example, when a polyethylene liquid impervious backsheet BS and a nonwoven layer (NW2) made of bicomponent polyethylene/polypropylene (sheath/core) filaments are used, the bonding of laminate AL with backsheet BS can be performed only by ultrasonic bonding or thermal bonding.

Manufacturing process

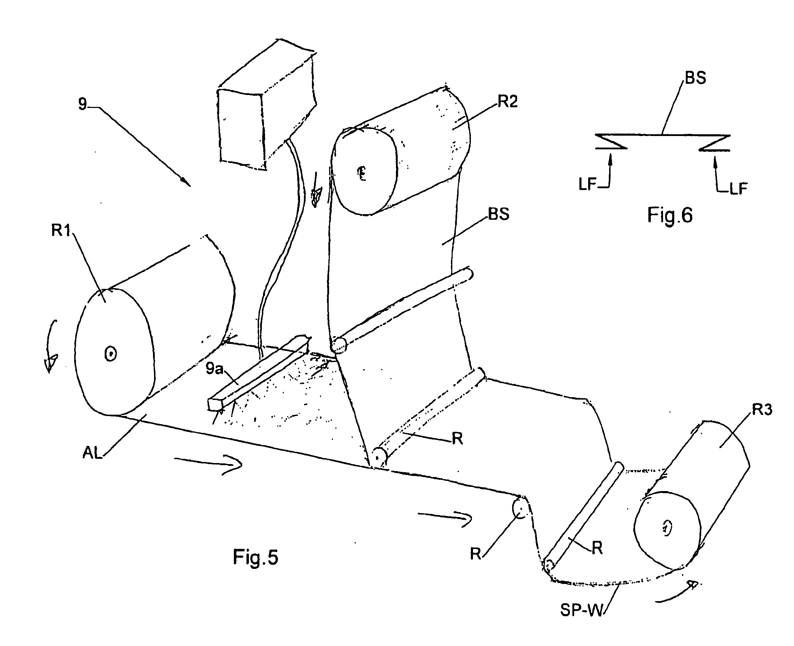

The absorbent surface pad SP is made in two stages. In a first stage, the absorbent laminate AL and the liquid impervious backsheet BS are produced off-line separately in the form of webs. In a second stage, the absorbent laminate web AL and the liquid impervious web BS are assembled together (figure 5).

The process for making a liquid impervious web BS is well known in the art and will not be described therein.

A preferred process for manufacturing the absorbent laminate web AL and a preferred process for making the absorbent pad from this absorbent laminate web and from a liquid impervious web BS are now going to be described.

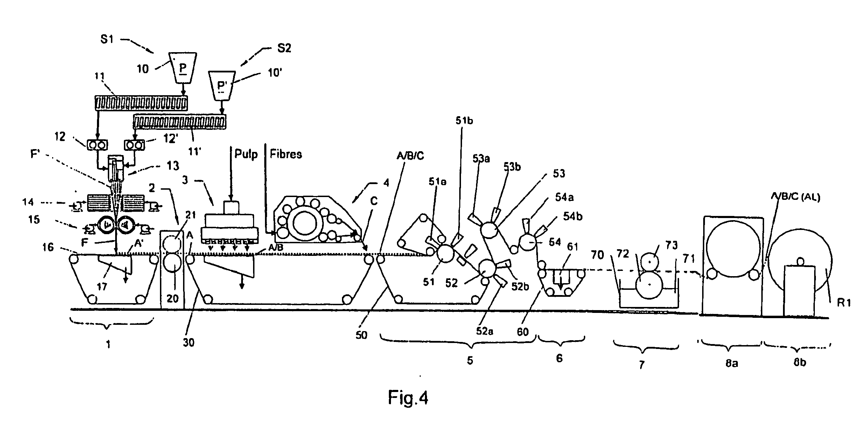

Manufacturing process of the absorbent laminate web (AL) / Figure 4

The absorbent laminate AL is advantageously manufactured by means of the continuous system of figure 4.

The continuous system of figure 4 comprises a spunbonding unit 1, a thermal bonding unit 2, an air-laid unit 3, a carding unit 4, a hydraulic needling unit 5, a dewatering unit 6, a hydrophilic treatment unit 7, a drying unit 8a, and a winding unit 8b.

Spunbonding unit 1

The spunbonding unit 1 is used for producing a non-consolidated spunbonded web A' made of continuous spun filaments F.

The spunbonding unit 1 comprises at least one supplying line S1. Said supplying line S1 comprises a feeding hopper 10, an extruder 11 and metering pumps 12. The feeding hopper 10 contains a polymeric material P

(for example in the form of pellets, or chips, or granulates,...). Said hopper 10 is connected to the inlet of the extruder 11, that enables to continuously heat up and molten the polymeric material P. The outlet of the extruder 11 is connected to the inlet of metering pumps 12, via a distribution manifold. The outlets of the metering pumps 12 are connected to the inlet of a spinning pack 13. The metering pumps 12 are used for continuously dosing the molten polymer P into the spinning pack 13. This spinning pack 13 is used for producing a curtain of continuous filaments F.

In the particular example of figure 1, the spunbonding unit 1 further comprises a second supplying line S2 for feeding the spinning pack 13 with a polymeric material P'. This second supplying line S2 comprises a feeding hopper 10', that contains the polymeric material P', an extruder 11', and metering pumps 12'.

Downstream the spinning pack 13, the spunbonding unit 1 comprises an air quenching box 14 that is being used to cool down the filaments F' issued from the spinning pack 13, and an air drawing equipment 15 that is being used to reduce the diameter of the filaments in order to form a curtain of filaments F having a smaller diameter.

The polymeric material(s) [P and/or P'] used for making the continuous spun filaments F can be any known spinnable polymeric material, and for example, polyolefin (in particular polypropylene or polyethylene), polyester, or polyamide, or any biodegradable thermoplastic polymer, like for example polylactic acid (PLA), or any blend thereof, or any copolymers thereof, or any blend of copolymers thereof.

These continuous spun filaments F can be, for example, monocomponent or multicomponent filaments, especially bicomponent filaments, and more especially sheath/core bicomponent filaments. When monocomponent spun filaments F are produced, only one supplying line (S1 or S2) can be used or both supplying lines can be used (S1 and S2). When bicomponent spun filaments F are produced, both supplying lines S1 and S2 are used simultaneously. In case of sheath/core bicomponent filaments, it is preferred, but not mandatory, to use polyethylene/polypropylene filaments.

Various shapes in cross section for the filaments F can also be envisaged (round shape, oval shape, bilobal shape, trilobal shape, etc...). The shape in cross section of the continuous spun filament F is determined by the geometry of the holes of the spinneret plate of the spinning pack 13.

The air drawing equipment 15 is mounted above a movable and foraminous surface, such as a wire conveyor belt 16. The spun filaments F of reduced diameter, that are issued from the air drawing equipment 15, are laid down onto the said movable surface 16, where vacuum is applied, opposite to the filaments lay down side, by means of a vacuum box 17. A non-consolidated spunbonded web A' made of continuous spun filaments F is thus formed on the surface of the belt 16, and is transported by the belt 16 towards the thermal bonding unit 2, that is mounted downstream the spunbonding unit 1.

Thermal bonding unit 2

Referring to figure 4, the spunbonded web A' is fed to a thermal bonding unit 2, that is being used in order to pre-consolidate the spunbonded web A' by heat and mechanical compression (thermo-bonding), and form the pre-consolidated spunbonded web A of the composite nonwoven of figure 1.

In the particular example of figure 2, said thermal bonding unit 2 is a calender that comprises two heated pressure rolls 20, 21. The lower roll has a smooth surface, and is for example a smooth steel roll. The upper roll 21 has an engraved surface with protruding ribs, that are regularly distributed over the whole surface of the roll, and that form a bonding pattern.

The heating temperature of said rolls 20, 21 is set up in order to obtain a softening of the surface of the filaments F. The mechanical pressure exerted by the rolls on the spunbonded web is sufficient in order to obtain a thermo-bonding of the spun filaments F, under heat and pressure.

Air-laid unit 3

The traditional air-laid unit 3, which is mounted downstream the thermal bonding unit 2, is disclosed in details, for example, in European patent application EP 0 032 772. Said air-laid unit 3 is fed with loose pulp fibers, and more preferably with short wood pulp fibers, but it can be fed also with a blend of loose pulp fibers and SAM and / or synthetic fibers.

The pre-consolidated spunbonded web A issued from thermal bonding unit 2 is transferred continuously to a second belt 30 where pulp fibers, or the above mentioned blend, are laid down, using a conventional air-laid process, by means of said traditional air-laid unit 3.

At the output of the air-laid unit 3, a composite (A/B) made of a pre-consolidated spunbonded web A and of an absorbent pulp top layer B is obtained.

Carding unit 4

The carding unit 4, which is mounted between the air-laid unit 3 and the hydraulic needling unit 5, is used for producing in line a carded nonwoven cover layer C. Said carded nonwoven cover layer C issued from the carding unit 4 is laid down onto the top surface of the absorbent pulp layer B of the composite nonwoven (A/B) issued from the air-laid unit 3.

Hydraulic needling unit 5

The composite nonwoven (A/B/C) is transported, downstream the carding unit 3, by means of a third conveyor belt 50 through the hydraulic needling unit 5. This hydraulic needling unit 5 is used for consolidating the nonwoven composite (A/B/C) and eventually to emboss it, by means of high pressure water jets (hydroentanglement process) that are directed at least towards the surface of the top layer (cover layer C), and that penetrate through the structure of the composite and are partially reflected back to the structure, in order to bind the layers (A, B and C) together.

In the particular example of figure 4, the water needling process is performed on both sides of the composite nonwoven (A/B/C).

More particularly, in the example of figure 4, the hydraulic needling unit 5 comprises four successive perforated drums. First perforated drum 51 is associated with two successive hydro-jet beams 51 a and 51b. Second perforated drum 52 is associated with two successive hydro-jet beams 52a and 52b. Third perforated drum 53 is associated with two successive hydro-jet beams 53a and 53b. The hydro-jet beam 53b can also be used to emboss one side of the nonwoven composite (ABC). Fourth perforated drum 54 is associated with two successive hydro-jet beams 54a and 54b. The hydro-jet beam 54b can also be used to emboss the other side of the nonwoven composite (ABC). The water pressure of the upstream hydro-jet beam 51a is lower than the water pressure of all the other downstream hydro-jet beams 51 b, 52a, 52b, 53a, 53b, 54a, 54b, in order to obtain a pre-hydroentanglement of the layers.

At the exit of hydraulic needling unit 5, a hydroentangled absorbent laminate web A/B/C with or without embossing is obtained.

Dewatering unit 6

This hydroentangled absorbent laminate web A/B/C is transported downstream the hydraulic needling unit 5 by the conveyor belt 60 of a dewatering unit 6, and over a vacuum box 61, that enables to remove by suction from the laminate A/B/C most of the water that has been absorbed during the water needling process (conventional dewatering process).

The hydroentanglement unit 5 and the dewatering unit 6 can be integrated in the same industrial equipment.

Hydrophilic treatment unit 7

The hydrophilic treatment unit is known in the art and comprises a tank 70 containing a hydrophilic solution 71, an applicator roll 72 in contact with the hydrophilic solution and a counter roll 73. The hydrophilic solution is made of a water soluble hydrophilic finish (for example 10wt%-20wt%) diluted in water (for example 90wt%-80wt%). By way of non limiting and non exhaustive examples, suitable candidates for the water soluble hydrophilic finish are notably water dispersable hydrophilic silicone finishes such as, for example, hydrophilic silicone "NuWet550®", commercialized by GE ADVANCED MATERIALS, and disclosed in US patent N° 5,811,482, hydrophilic finish "DURON OF 4012 VP" commercialized by CHT, hydrophilic finish "Stantex S-6327" commercialized by COGNIS, hydrophilic finish "Silastol 163" commercialized by SCHILL + SEILACHER, hydrophilic finish "Delion NW-291" commercialized by TAKEMOTO.

The laminate A/B/C passes between the applicator roll 72 and the counter roll 73, the spunbonded layer A being in contact with the applicator roll 72. During its rotation, the applicator roll 72 takes off hydrophilic solution from the tank 70 and coats the spunbonded layer A with the hydrophilic solution. Downstream the hydrophilic treatment unit 7, the spunbonded layer A of laminate A/B/C is thus advantageously hydrophilic.

In another variant of the invention, the hydrophilic treatment unit can be modified knowingly by one skilled in the art for applying a hydrophilic finish on the bottom layer C (carded layer or spunbonded layer) of the laminate or for applying a hydrophilic finish on both sides of the laminate, i.e. on top layer A (spunbonded layer) and on bottom layer C(carded layer or spunbonded layer)

Drying unit 8a

The hydroentangled absorbent laminate web A/B/C issued from the hydrophilic treatment unit 7 is continuously fed through the oven of the drying unit 8, wherein heat is applied to the composite (for example by means of hot air), in order to remove the remaining water still contained within the web.

The absorbent laminate AL of the absorbent pad SP of figure 1 is obtained from the said dried composite laminate web A/B/C, the layer B being the absorbent pulp layer PL of the laminate AL of figure 1, the spunbonded layer A being the liquid permeable and hydrophilic layer NW1 and the carded layer C being the liquid permeable layer NW2.

Winding unit 8b

The absorbent laminate web AL (A/B/C) is wound in the form of a roll R1, by means of the winding unit 8.

Manufacturing process of the absorbent surface pad (SP) / Figure 5

Referring to figure 5, an absorbent pad web SP-W is made from a roll R1 of absorbent laminate web AL and from a roll R2 of a liquid impervious web BS.

The absorbent laminate web AL is unwounded and hot melt glue G is continuously sprayed on the top surface of the absorbent laminate web AL, by means of spraying unit 9 comprising a spraying nozzle 9a.

The liquid impervious web BS is unwound and laid onto the glued absorbent laminate web AL downstream the spraying nozzle 9a. The two webs AL and BS are pressed together by means of rolls R, in such a way that the liquid impervious web BS adheres firmly to the absorbent laminate web AL.

Then, in the particular example figure 5, the web SP-W formed by the two webs AL and BS is wound in the form of a roll R3.

In order to form the absorbent pads SP having the rectangular or square form of figure 2, the web SP-W is unwound and cut afterwards in the cross direction, and if necessary in the machine direction, in order to form single surface pads SP. In another variant, these cutting operations can be also performed in line with the assembling operation of the two webs AL and BS, instead of winding the web SP-W (roll R3).

In order to manufacture the absorbent pad SP of figure 3, roll R2 has to be replaced by a roll of a liquid impervious web BS, whose longitudinal edges are folded, as depicted on figure 6, in order to form the lateral flap LF.

Within the scope of the invention, the web of absorbent laminate AL is not necessarily manufactured off-line, but in another variant, the web of absorbent laminate AL can be also manufactured and assembled in line with the web forming the backsheet BS. The liquid impervious web forming the backsheet BS can be also manufactured in line with the web of absorbent laminate AL.

Some advantages of the invention will now be illustrated by the following non-limiting tests. These tests were performed:

> (i) on bed pad samples of the prior art, referred "P1", "P2", "P3" and "P4" in the following tables A1, A2, I, II, III, IV
> (ii) on an absorbent bed pad (SP) of the invention.

Product P1 is a bed pad commercialized by ARTSANA under trademark "Serenity®". Product P2 is a bed pad commercialized by SCA under trademark "Tena Bed® Plus". Product P3 is a bed pad commercialized by SILC Spa under trademark "SOFFISOFT®". Product P4 is a bed pad commercialized by FATER Spa under trademark "LINIDOR®".

The main characteristics of products P1 to P4 are summarized in tables A1 and A2.

The structure of products P1 to P4 (prior art) comprises a liquid permeable spunbonded nonwoven topsheet (TS) and a liquid impervious polyethylene (PE) backsheet (BS) that are glued together, and an absorbent fluff core layer (CORE) positioned between the said topsheet (TS) and backsheet (BS). Product P2 further comprises a wet resistant (WR) tissue (TISSUE) in between the topsheet (TS) and the absorbent fluff core layer (CORE).

## Table A1 : Products P1 to P4

| PRODUCT | SIZE | Number of pads per pack | PACK DIMENSIONS [cm] / VOLUME [cm³] | PAD TOTAL WEIGHT [g] | PAD DIMENSIONS length x width [mm] | CORE DIMENSIONS length x width [mm] | CORE AREA COVERAGE % |
|---|---|---|---|---|---|---|---|
| P4 | MEDIUM 60x90 cm | 30 | - | 69.0 | 600x902 | 550x825 | 83.8 |
| P1 | MEDIUM 60x90 cm | 30 | 30 x 43 x 16 / 20640 | 95.7 | 600 x 910 | 540 x 780 | 77.1 |
| P2 | (PLUS) MEDIUM 60x90 cm | 30 | 29 x 40 x 20 / 23200 | 98.0 | 600 X 920 | 560 X 830 | 84.2 |
| P3 | MEDIUM 60x90 cm | 15 | 24 x 30 x 15 / 10800 | 103.4 | 610 x 910 | 540 x 820 | 79.8 |

## Table A2

| PRODUCT | STRUCTURE | TS TYPE DIMENS. l x w [mm] | TISSUE TYPE DIMENS. l x w [mm] | CORE TYPE DIMENS. l x w [mm] | BS TYPE DIMENS. l x w [mm] | TS Total Weight [g] BW [gsm] | TISSUE Total Weight [g] BW [gsm] | CORE Total Weight [g] BW [gsm] | BS Total Weight [g] BW [gsm] | GLUE [g] BW [gsm] |
|---|---|---|---|---|---|---|---|---|---|---|
| P4 | TS + CORE + BS | SPUNB. 600x902 | | CELL. FLUFF 550 x 825 | PE 660x902 | 7.6 / 14 | . | 47.6 / 105 | 11.3 / 19 | 2.5 / 4.2 |
| P1 | TS + CORE + BS | SPUNB. 600x910 | | CELL. FLUFF 540 x 780 | PE 600 x 910 | 8.2 / 15 | | 68.2 / 162 | 16.5 / 30 | 2.8 / 5.1 |
| P2 | TS + TISSUE + CORE + BS | SPUNB. 600x920 | WR 560x830 | CELL. FLUFF 560 x 830 | PE 650 x 920 | 7.7 / 14 | 8.8 / 19 | 66.0 / 142 | 13.2 / 22 | 3 / 5 |
| P3 | TS + CORE + BS | SPUNB. 610x910 | | CELL. FLUFF 540 x 820 | PE 610 x 910 | 8.9 / 16 | | 78.9 / 175 | 13.9 / 25 | 2.7 / 4.9 |

The main characteristics of the bed pad (SP) of the invention that were used for the test are summarized in tables B1 and B2.

## Table B1 :

| PRODUCT | SIZE | Number of pads per pack | PACK DIMENSIONS [cm] / VOLUME [cm³] | PAD TOTAL WEIGHT [g] | PAD DIMENSIONS length x width [mm] | CORE DIMENSIONS length x width [mm] | CORE AREA COVERAGE % |
|---|---|---|---|---|---|---|---|
| SP | 60x90 cm | 30 | 19x28x18 / 9576 | 48.7 | 600x902 | 600x902 | 100 |

### Table B2 :

| PRODUCT | STRUCTURE | AL<br><br>DIMENS.<br>l x w<br>[mm] | BS<br><br>DIMENS.<br>l x w<br>[mm] | AL<br><br><br>Total weight<br>[g] | AL<br>(NW1/PL/NW2)<br><br>Basic weight<br>[gsm] | BS<br>Total<br>Weight<br>[g]<br>BW<br>[gsm] | GLUE<br>[g]<br><br><br>BW<br>[gsm] |
|---|---|---|---|---|---|---|---|
| SP | AL<br>(NW1/PL/<br>NW2)[1]<br>+<br>BS | 600x902 | 660x902 | 37.9 | 70<br>(10/45/15) | 10.3<br><br>19 | 0.5<br><br>0.9 |

(1) AL [NW1: Spunbonded layer / PL: Pulp layer/ NW2: Carded layer] - Hydroentangled

Test N°1/ Product thickness

The thickness of the bed pads were measured according to standard method WSP 120.6 [05]. The area of pressure foot was 25cm$^2$. The measurements were performed at two different loading pressures: 0.5KPa and 2.1 KPa. The results of the thickness measurements are given in table I below.

Table I :

| PRODUCT THICKNESS expressed in mm<br>(Number of samples = 10) | | | | | |
|---|---|---|---|---|---|
| Bed Pads | P1 | P2 | P3 | P4 | Invention (SP) |
| Dimensions (cmxcm) | 60X90 | 60X90 | 60X90 | 60x90 | 60x90 |
| **0.5 KPa** | 2.92 | 2.87 | 2.98 | 2.16 | **0.67** |
| Standard Deviation | 1.116 | 0.45 | 0.486 | 0.445 | **0.038** |
| **2.1 KPa** | 2.4 | 1.75 | 2.41 | 2.15 | 0.57 |
| Standard Deviation | 0.88 | 0.31 | 0.3 | 0.45 | 0.026 |

The bed pad (SP) of the invention is advantageously very thin (thickness less than 1mm at 0.5KPa), compared with products of the prior art P1 to P4.

Test N°2 / MD Tensile strength

The tensile strength measurements in the machine direction (MD) were performed on a dynamometer model 5564 available from Instron Instruments, according to WSP 110.4 (05) standard. Specimens were cut 25.4 mm width and at least 150mm length. The traction speed was set at 300mm/min.
The results of the MD tensile strength measurements are given in table II below.

Table II:

| TENSILE STRENGTH expressed in N/cm (Number of samples = 10) | | | | | |
|---|---|---|---|---|---|
| Bed Pads | P1 | P2 | P3 | P4 | Invention (SP) |
| Dimensions (cmxcm) | 60X90 | 60X90 | 60X90 | 60x90 | 60x90 |
| MD | 13.70 | 9.19 | 12.5 | 13.00 | **16.41** |
| Standard Deviation | 0.58 | 1.01 | 0.66 | 1.92 | **0.50** |

The MD tensile strength of the bed pad (SP) of the invention is advantageously higher. The bed pad (SP) of the invention has thereby a longer product life during usage.

Test N°3 / Average wetted diameter and Average wetted area

The objective of this test was to evaluate the liquid distribution capability of the bed pad of the invention (SP) compared with the liquid distribution capability of products P2 and P4, and by applying the following procedure: a 10 ml of physiological saline solution (0.9% sodium chloride), blue colored, were injected from 10 cm distance from top to bottom in a single point, centered on the bed pad ; the average diameter of the blue stain (wetted area) after 1 minute from injection was then measured. The results of these measurements are given in table III below.

Table III

| AVERAGE WETTED DIAMETER/AREA | | | |
|---|---|---|---|
| Bed Pads | P4 | **Invention (SP)** | P2 |
| Dimensions (cmxcm) | 60X90 | 60X90 | 60X90 |
| Average stain diameter (cm) | 10 | **15.5** | 9 |
| Average stain area (cm$^2$) | 78.5 | **188.6** | 63.6 |

We obtained a larger blue stain (i.e. wetted area) with the bed pad (SP) of the invention. The liquid distribution capability is thus improved. This is also demonstrated by appended figure 7 (photography of bed pad P4 showing the blue stain obtained after 1 minute) compared with figure 8 (photography of the bed pad (SP) of the invention and showing the larger and more uniform blue stain obtained after 1 minute).

Test N°4/ Dryness

The objective of this test was to evaluate the dryness property of the bed pad of the invention (SP) compared with the dryness property of products P2 and P4, and by applying the following method referred therein and in the claims as "rewet method".

Rewet method:

We inject on the pad 10 ml of blue colored physiological saline solution (0.9% sodium chloride), from 10 cm distance in a single point, centered on the bed pad ; we wait for 30 minutes, then we place 5 filters type H&V-ERTMWWSSHEETS/125mmx125mm centered above the wetted area, and we load the filters with 3.6 Kg weight centered onto the 5 filters for further 3 minutes. The 3.6 Kg weight has the following dimensions: 100mmx100mm and the side in contact to the filters is constituted by a 25 microns PE film. Between the PE film and the 3,6 Kg weight is interposed a PU foam having 20 mm thickness. The liquid coming back from the pad wets the filters and the weight of the wetted filters is measured.
The rewet (r) is given by the following formula:

$$r = W2 - W1,$$

Wherein:

W1 is the weight of the non-wetted filters
W2 is the weight of the wetted filters after 3 minutes under 3,6Kg loading.

The results of these measurements are given in table IV below.

Table IV :

| DRYNESS EVALUATION TEST | | | |
|---|---|---|---|
| Bed pads | P4 | **Invention (SP)** | P2 |
| Dimensions (cmxcm) | 60X90 | 60X90 | 60X90 |
| Rewet r (g of liquid) | 6.63 | **2.5** | 5.51 |

The bed pad (SP) of the invention exhibits a lower rewet, which means a better dryness for the user adult incontinent lying on the wetted bed pad.

**Claims**

1.  A liquid absorbent surface pad (SP) comprising liquid absorbent means and a liquid impervious backsheet, the liquid absorbent means being made of an absorbent laminate (AL) that is bonded with the liquid impervious backsheet (BS), **characterized in that** the absorbent laminate comprises at least three hydroentangled layers : one pulp layer (PL) sandwiched between two liquid permeable nonwoven layers (NW1; NW2), at least one of the two liquid permeable nonwoven layers (NW1; NW2) being a spunbonded nonwoven layer, and the other liquid permeable nonwoven layer being a spunbonded nonwoven layer or a carded nonwoven layer.

2.  A liquid absorbent surface pad according to claim 1, wherein the laminate is constituted by the said three layers.

3.  A liquid absorbent surface pad according to claim 1 or 2, wherein at least the top nonwoven layer (NW1), which is not in contact with the liquid impervious backsheet (BS), is hydrophilic.

4.  A liquid absorbent surface pad according to any one of claims 1 to 3, wherein the absorbent laminate (AL) is glued with the liquid impervious backsheet (BS).

5.  A liquid absorbent surface pad according to any one of claims 1 to 4, and constituted by the said hydroentangled absorbent laminate (AL) and the said liquid impervious backsheet (BS).

6.  A liquid absorbent surface pad according to any one of claims 1 to 5, wherein the basis weight of the pulp layer (PL) is less than 100gsm.

7.  A liquid absorbent surface pad according to claim 6, wherein the basis weight of the pulp layer (PL) is less than 60gsm.

8.  A liquid absorbent surface pad according to any one of claims 1 to 7, having a thickness less than 1mm measured at a loaded pressure of 0.5KPa.

9.  A liquid absorbent pad according to any one of claims 1 to 8 having a machine direction tensile strength, measured accordingly to WSP 110.4 (05) standard method, that is higher than 14 N/cm, and preferably higher than 16 N/cm.

10.  A liquid absorbent pad according to any one of claims 1 to 9 having a rewet less than 3g of liquid measured according to the so-called "rewet method" whose steps are described in the description.

11.  A liquid absorbent pad according to any one of claims 1 to 10 and constituting a bed pad.

12.  A method of making liquid absorbent surface pads (SP), comprising the following steps:

    - providing a web of an absorbent laminate web (AL) that comprises at least three layers bonded together by hydroentangling : one pulp layer (PL) sandwiched between two liquid permeable nonwoven layers (NW1; NW2), and at least one of the two liquid permeable nonwoven layers (NW1; NW2) is a spunbonded nonwoven layer, and the other liquid permeable nonwoven layer is a spunbonded nonwoven layer or a carded nonwoven layer,
    - providing a liquid impervious backsheet web (BS),
    - bonding together the absorbent laminate web (AL) and the liquid impervious backsheet web (BS),
    - cutting the web constituted by the absorbent laminate web (AL) and by the liquid impervious backsheet web (BS) into single absorbent pads (SP).

13.  A method according to claim 12, wherein the absorbent laminate web (AL) and the liquid impervious backsheet web (BS) are manufactured off-line separately.

14.  A method according to any one of claims 12 to 13, wherein the absorbent laminate web (AL) is glued with the liquid impervious backsheet web (BS).

15.  A method according to any one of claims 12 to 14, wherein the absorbent laminate web (AL) is ultrasonic bonded or thermal bonded with the liquid impervious backsheet web (BS).

16. A method according to any one of claims 12 to 15, wherein the basis weight of the pulp layer (PL) of the absorbent laminate web (AL) is less than 100gsm.

17. A method according to claim 16, wherein the basis weight of the pulp layer (PL) is less than 60gsm.

18. A method according to any one of claims 12 to 17 wherein the thickness of the web made from the absorbent laminate web (AL) and from the liquid impervious backsheet web (BS) is less than 1 mm measured at a loaded pressure of 0.5KPa.

19. Use of the liquid absorbent surface pad (SP) of any one of claims 1 to 11 as bed pad.


**Patentansprüche**

1. **Pad oder Auflage mit flüssigkeitsabsorbierender Oberfläche** (SP), mit einem flüssigkeitsabsorbierenden Mittel und einer flüssigkeitsundurchlässigen Rückseite, wobei das flüssigkeitsabsorbierende Mittel aus einem absorbierenden Schichtstoff (AL) besteht, der mit der flüssigkeitsundurchlässigen Rückseite (BS) verbunden ist, **dadurch gekennzeichnet, dass** der absorbierende Schichtstoff zumindest drei wasserstrahlvernadelte oder wasserstrahlverfestigte Schichten umfasst: eine Zellstoffschicht (PL) zwischen zwei flüssigkeitsdurchlässigen Vliesschichten (NW1; NW2), wobei zumindest eine der beiden flüssigkeitsdurchlässigen Vliesschichten (NW1; NW2) eine Spinnvliesschicht ist und die andere flüssigkeitsdurchlässige Vliesschicht eine Spinnvliesschicht oder eine kardierte Vliesschicht ist.

2. Pad mit flüssigkeitsabsorbierender Oberfläche nach Anspruch 1, wobei der Schichtstoff aus den drei Schichten gebildet ist.

3. Pad mit flüssigkeitsabsorbierender Oberfläche nach einem der Ansprüche 1 oder 2, wobei zumindest die obere Vliesschicht (NW1), die die flüssigkeitsundurchlässige Rückseite (BS) nicht kontaktiert, hydrophil ist.

4. Pad mit flüssigkeitsabsorbierender Oberfläche nach einem der Ansprüche 1 bis 3, wobei der absorbierende Schichtstoff (AL) mit der flüssigkeitsundurchlässigen Rückseite (BS) verklebt ist.

5. Pad mit flüssigkeitsabsorbierender Oberfläche nach einem der Ansprüche 1 bis 4, gebildet durch den wasserstrahlverfestigten oder wasserstrahlvernadelten absorbierenden Schichtstoff (AL) und die flüssigkeitsundurchlässige Rückseite (BS).

6. Pad mit flüssigkeitsabsorbierender Oberfläche nach einem der Ansprüche 1 bis 5, wobei das Grundgewicht der Zellstoffschicht (PL) weniger als 100 gsm beträgt.

7. Pad mit flüssigkeitsabsorbierender Oberfläche nach Anspruch 6, wobei das Grundgewicht der Zellstoffschicht (PL) weniger als 60 gsm beträgt.

8. Pad mit flüssigkeitsabsorbierender Oberfläche nach einem der Ansprüche 1 bis 7 mit einer Dicke von weniger als 1 mm, gemessen bei einem Belastungsdruck von 0,5 kPa.

9. Pad mit flüssigkeitsabsorbierender Oberfläche nach einem der Ansprüche 1 bis 8 mit einer Reißfestigkeit in Maschinenrichtung, gemessen nach der Standardmethode WSP 110.4 (05), größer als 14 N/cm, und bevorzugt größer als 16 N/cm.

10. Pad mit flüssigkeitsabsorbierender Oberfläche nach einem der Ansprüche 1 bis 9 mit einer Rücknässe (rewet) von weniger als 3 g Flüssigkeit, gemessen nach der sogenannten "Rücknässungsmethode" ("rewet method"), dessen Schritte in der Beschreibung beschrieben sind.

11. Pad mit flüssigkeitsabsorbierender Oberfläche nach einem der Ansprüche 1 bis 10, wodurch ein Bett-Pad oder eine Bettauflage gebildet wird.

**12. Verfahren** zum Herstellen von Pads mit flüssigkeitsabsorbierender Oberfläche (SP), mit den folgenden Schritten:

- Bereitstellen einer Bahn einer absorbierenden Schichtstoffbahn (AL), die zumindest drei durch Wasserstrahlverfestigung oder Wasserstrahlvernadlung verbundene Schichten umfasst: eine Zellstoffschicht (PL) zwischen zwei flüssigkeitsdurchlässigen Vliesschichten (NW1; NW2), wobei zumindest eine der beiden flüssigkeitsdurchlässigen Vliesschichten (NW1; NW2) eine Spinnvliesschicht ist und die andere flüssigkeitsdurchlässige Vliesschicht eine Spinnvliesschicht oder eine kardierte Vliesschicht ist,
- Bereitstellen einer flüssigkeitsundurchlässigen Rückseitenbahn (BS),
- Verbinden der absorbierenden Schichtstoffbahn (AL) und der flüssigkeitsundurchlässigen Rückseitenbahn (BS),
- Schneiden der Bahn, gebildet durch die absorbierende Schichtstoffbahn (AL) und die flüssigkeitsundurchlässige Rückseitenbahn (BS) in einzelne absorbierende Pads (SP).

**13.** Verfahren nach Anspruch 12, wobei die absorbierende Schichtstoffbahn (AL) und die flüssigkeitsundurchlässige Rückseitenbahn (BS) außerhalb der Linie separat hergestellt werden.

**14.** Verfahren nach einem der Ansprüche 12 bis 13, wobei die absorbierende Schichtstoffbahn (AL) mit der flüssigkeitsundurchlässigen Rückseitenbahn (BS) verklebt ist.

**15.** Verfahren nach einem der Ansprüche 12 bis 14, wobei die absorbierende Schichtstoffbahn (AL) mit der flüssigkeitsundurchlässigen Rückseitenbahn (BS) ultraschallverschweißt oder thermisch verschweißt ist.

**16.** Verfahren nach einem der Ansprüche 12 bis 15, wobei das Grundgewicht der Zellstoffschicht (PL) der absorbierenden Schichtstoffbahn (AL) weniger als 100 gsm beträgt.

**17.** Verfahren nach Anspruch 16, wobei das Grundgewicht der Zellstoffschicht (PL) weniger als 60 gsm beträgt.

**18.** Verfahren nach einem der Ansprüche 12 bis 17, wobei die Dicke der Bahn, die aus der absorbierenden Schichtstoffbahn (AL) und der flüssigkeitsundurchlässigen Rückseitenbahn (BS) hergestellt ist, weniger als 1 mm beträgt, gemessen bei einem Belastungsdruck von 0,5 kPa.

**19. Verwendung** des Pads mit flüssigkeitsabsorbierender Oberfläche (SP) nach einem der Ansprüche 1 bis 11 als Bett-Pad oder Bettauflage.

**Revendications**

**1.** Article à surface absorbante de liquide (SP) comprenant des moyens d'absorption de liquide et une feuille arrière imperméable aux liquides, les moyens d'absorption de liquide étant constitués d'un stratifié absorbant (AL) qui est lié à la feuille arrière imperméable aux liquides (BS), **caractérisé en ce que** le stratifié absorbant comprend au moins trois couches hydro-enchevêtrées: une couche de pâte à pulpe(PL) prise en sandwich entre deux couches non tissées perméables aux liquides (NW1; NW2), au moins une des deux couches non tissées perméables aux liquides (NW1; NW2) étant une couche non tissée filée-liée, et l'autre couche non tissée perméable aux liquides étant une couche non tissée filée-liée ou une couche non tissée cardée.

**2.** Article à surface absorbante de liquide selon la revendication 1, dans lequel le stratifié est constitué par lesdites trois couches.

**3.** Article à surface absorbante de liquide selon la revendication 1 ou 2, dans lequel au moins la couche non tissée supérieure (NW1), qui n'est pas en contact avec la feuille arrière imperméable aux liquides (BS), est hydrophile.

**4.** Article à surface absorbante de liquide selon l'une quelconque des revendications 1 à 3, dans lequel le stratifié absorbant (AL) est collé à la feuille arrière imperméable aux liquides (BS).

**5.** Article à surface absorbante de liquide selon l'une quelconque des revendications 1 à 4, et constitué par ledit stratifié absorbant hydro-enchevêtré (AL) et ladite feuille arrière imperméable aux liquides (BS).

**6.** Article à surface absorbante de liquide selon l'une quelconque des revendications 1 à 5, dans lequel le poids de base de la couche de pâte à pulpe(PL) est inférieur à 100 g/m$^2$.

7. Article à surface absorbante de liquide selon la revendication 6, dans lequel le poids de base de la couche de pâte à pulpe(PL) est inférieur à 60 g/m$^2$.

8. Article à surface absorbante de liquide selon l'une quelconque des revendications 1 à 7, présentant une épaisseur inférieur à 1 mm mesurée à une pression de charge de 0,5 kPa.

9. Article à surface absorbante de liquide selon l'une quelconque des revendications 1 à 8, présentant une résistance à la traction dans le sens machine, mesurée par un procédé standard WSP 110.4 (05), qui est supérieure à 14 N/cm, et de préférence supérieure à 16 N/cm.

10. Article à surface absorbante de liquide selon l'une quelconque des revendications 1 à 9, présentant une ré-humidification inférieure à 3 g de liquide mesurée selon ce que l'on appelle le "procédé de ré-humidification" dont les étapes sont décrites dans la description.

11. Article à surface absorbante de liquide selon l'une quelconque des revendications 1 à 10 et constituant une alèse.

12. Procédé de fabrication d'articles à surface absorbante de liquide (SP), comprenant les étapes suivantes:

    - fournir une toile d'une toile de stratifié absorbant (AL) comprenant au moins trois couches liées ensemble par hydro-enchevêtrement: une couche de pâte à pulpe(PL) prise en sandwich entre deux couches non tissées perméables aux liquides (NW1; NW2), au moins une des deux couches non tissées perméables aux liquides (NW1; NW2) étant une couche non tissée filée-liée, et l'autre couche non tissée perméable aux liquides étant une couche non tissée filée-liée ou une couche non tissée cardée;
    - fournir une toile de feuille arrière imperméable aux liquides (BS);
    - lier ensemble la toile de stratifié absorbant (AL) et la toile de feuille arrière imperméable aux liquides (BS); et
    - découper la toile constituée par la toile de stratifié absorbant (AL) et par la toile de feuille arrière imperméable aux liquides (BS) en articles absorbants individuels (SP).

13. Procédé selon la revendication 12, dans lequel la toile de stratifié absorbant (AL) et la feuille arrière imperméable aux liquides (BS) sont fabriquées hors ligne séparément.

14. Procédé selon l'une quelconque des revendications 12 à 13, dans lequel la toile de stratifié absorbant (AL) est collée à la feuille arrière imperméable aux liquides (BS).

15. Procédé selon l'une quelconque des revendications 12 à 14, dans lequel la toile de stratifié absorbant (AL) est liée aux ultrasons ou est liée thermiquement à la toile de feuille arrière imperméable aux liquides (BS).

16. Procédé selon l'une quelconque des revendications 12 à 15, dans lequel le poids de base de la couche de pâte à pulpe(PL) de la toile de stratifié absorbant (AL) est inférieur à 100 g/m$^2$.

17. Procédé selon la revendication 16, dans lequel le poids de base de la couche de pâte à pulpe(PL) est inférieur à 60 g/m$^2$.

18. Procédé selon l'une quelconque des revendications 12 à 17, dans lequel l'épaisseur de la toile constituée de la toile de stratifié absorbant (AL) et de la feuille arrière imperméable aux liquides (BS) est inférieure à 1 mm, mesurée à une pression de charge de 0,5 kPa.

19. Utilisation de l'article à surface absorbante de liquide (SP) selon l'une quelconque des revendications 1 à 11 comme alèse.

Fig.1

AL/BS

SP

M

Fig.2

AL/BS

SP

BS

M

LF

LF

Fig.3

Fig.4

19

Fig.5

Fig.6

EP 2 166 998 B1

Fig.7

Fig.8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4844695 A
- US 20010031515 A
- EP 0556996 A
- EP 0032772 A
- US 5811482 A